(19) Europäisches Patentamt
European Patent Office
Office européen des brevets

(11) **EP 4 058 792 B1**

(12) **EUROPÄISCHE PATENTSCHRIFT**

(45) Veröffentlichungstag und Bekanntmachung des Hinweises auf die Patenterteilung:
**16.04.2025   Patentblatt 2025/16**

(21) Anmeldenummer: **20807348.6**

(22) Anmeldetag: **13.11.2020**

(51) Internationale Patentklassifikation (IPC):
**G01N 27/12** (2006.01)    **G01N 33/00** (2006.01)

(52) Gemeinsame Patentklassifikation (CPC):
**G01N 27/124; G01N 33/0006; G01N 33/007**

(86) Internationale Anmeldenummer:
**PCT/EP2020/082051**

(87) Internationale Veröffentlichungsnummer:
**WO 2021/094529 (20.05.2021 Gazette 2021/20)**

(54) **VERFAHREN UND VORRICHTUNG ZUM BESTIMMEN EINER DEGRADATION EINES HALBLEITERGASSENSORS**

METHOD AND APPARATUS FOR DETERMINING A DEGRADATION OF A SEMICONDUCTOR GAS SENSOR

PROCÉDÉ ET APPAREIL DE DÉTERMINATION DE DÉGRADATION D'UN CAPTEUR DE GAZ À SEMI-CONDUCTEUR

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **15.11.2019   DE 102019130990**

(43) Veröffentlichungstag der Anmeldung:
**21.09.2022   Patentblatt 2022/38**

(73) Patentinhaber: **Universität des Saarlandes 66123 Saarbrücken (DE)**

(72) Erfinder:
- **SAUERWALD, Tilman**
  **66123 Saarbrücken (DE)**
- **SCHÜTZE, Andreas**
  **66386 St. Ingbert (DE)**
- **SCHULTEALBERT, Caroline**
  **66111 Saarbrücken (DE)**
- **BAUR, Tobias**
  **66111 Saarbrücken (DE)**
- **UZUN, Iklim**
  **66333 Völklingen (DE)**

(74) Vertreter: **Banse & Steglich Patentanwälte PartmbB Patentanwaltskanzlei Herzog-Heinrich-Straße 23 80336 München (DE)**

(56) Entgegenhaltungen:
DE-T2- 69 837 965

- **M. SCHÜLER ET AL: "A novel approach for detecting HMDSO poisoning of metal oxide gas sensors and improving their stability by temperature cycled operation", JOURNAL OF SENSORS AND SENSOR SYSTEMS, vol. 4, no. 2, 19 October 2015 (2015-10-19), pages 305 - 311, XP055727572, DOI: 10.5194/jsss-4-305-2015**
- **M. SCHÜLER ET AL: "E8.4 - Detecting poisoning of metal oxide gas sensors at an early stage by temperature cycled operation", PROCEEDINGS SENSOR 2015, 21 May 2015 (2015-05-21), XP055727229, ISBN: 978-3-9813484-8-4, DOI: 10.5162/sensor2015/e8.4**
- **TOBIAS BAUR ET AL: "Optimierung des temperaturzyklischen Betriebs von Halbleitergassensoren", TM - TECHNISCHES MESSEN, vol. 82, no. 4, 1 April 2015 (2015-04-01), pages 187 - 195, XP009525139, ISSN: 2196-7113, DOI: 10.1515/TEME-2014- 0007**

## EP 4 058 792 B1

**Beschreibung**

Technisches Gebiet

Die Erfindung betrifft Gassensoren, insbesondere Halbleitergassensoren, zur Messung von Gaskonzentrationen von Gaskomponenten in einem Messgas. Die Erfindung betrifft weiterhin Verfahren zum Bestimmen einer Degradation von Eigenschaften einer aktiven Sensorfläche des Gassensors und Korrigieren einer Sensorgröße eines Halbleitergassensors basierend auf einer solchen Degradation.

Technischer Hintergrund

Die Stabilität herkömmlicher chemischer Sensoren mit aktiver Sensorfläche ist aufgrund von Umwelteinflüssen und Alterungserscheinungen begrenzt. So nimmt die Detektionsempfindlichkeit von Halbleitergassensoren im Laufe der Lebensdauer aufgrund einer Degradation der aktiven Sensorfläche ab. Maßgeblich tragen zu einer solchen Degradation sogenannte Sensorgifte bei, die auf der aktiven Sensorfläche dauerhaft Oberflächenplätze besetzen und blockieren und auch bei hohen Temperaturen nicht mehr aktiviert bzw. desorbiert werden können.

Beispielsweise stellen Siloxane, Halogenverbindungen, phosphatbildende Verbindungen und Schwefelverbindungen derartige Sensorgifte dar. Insbesondere sind organische Silikone Sensorgifte. Diese sind weit verbreitet und finden z. B. als Elastomere, Schnürmittel, Entschäumungsmittel sowie in Kosmetika und Farben Verwendung. Somit ist bei üblichen Einsatzgebieten von chemischen Gassensoren mit dem Auftreten von organischen Silikonen zu rechnen, im Besonderen bei der Messung der Innenraum-Luftqualität und der Messung von Gerüchen sowie bei medizinischen Anwendungen.

Der Nachweis der Beständigkeit von Luftschadstoffsensoren gegenüber organischen Silikonverbindungen wird derzeit in einigen Normen und technischen Vorschriften verlangt, z. B. für die Messung der Innenraumluftqualität (ISO 16000-29) sowie in der Messung von explosiven Gasen in Wohnräumen (DIN EN 50194-1).

Bislang wird die Beständigkeit über eine große Zahl an aktiven Oberflächenplätzen der Sensorfläche realisiert, insbesondere durch einen deutlichen Überschuss an katalytisch aktivem Material. Durch die während der Lagerung oder Alterung angebotene Dosis wird sich dann die Aktivität der Sensoroberflächen nur wenig ändern, so dass die Sensorreaktion im Rahmen der spezifizierten Genauigkeit bleibt. Jedoch sind in vielen Anwendungsszenarien deutlich höhere Beaufschlagungen von Gassensoren mit Sensorgiften zu erwarten, was gegebenenfalls nicht mehr durch die Auslegung des Gassensors kompensierbar ist.

Insbesondere können Multisensorsysteme von der Degradation durch Umwelteinflüsse und Alterung besonders betroffen sein, da Messwerte häufig durch Verrechnung von Messsignalen mehrerer Gassensoren gewonnen werden. Signaländerungen aufgrund Degradation können sich hierbei sehr verstärken, wenn z. B. der Messwert durch eine gewichtete Differenz mehrerer Signalsysteme ermittelt wird. Multisignalsysteme werden häufig verwendet, um Konzentrationen spezifischer Gase zu messen und Querempfindlichkeiten zu unterdrücken.

Halbleitergassensoren werden häufig in einem temperaturzyklischem Betrieb betrieben, wobei in einem ersten Schritt einer Hochtemperaturphase die aktive Sensorfläche aus einem thermodynamischen Gleichgewicht gebracht wird und in einem nachfolgenden zweiten Schritt die aktivierte Sensorfläche während einer Messphase bei geringerer Temperatur zur Messung einer Gaskonzentration verwendet wird. Das Maß der Aktivierung der Sensorfläche in der Hochtemperaturphase bestimmt die Empfindlichkeit des Gassensors. Die Aktivierbarkeit der Sensorfläche hängt jedoch maßgeblich von der alterungs- und lagerungsbedingten Degradation der Sensorfläche ab. Daher ist eine Kalibrierung bzw. Nachjustierung eines solchen Gassensors regelmäßig notwendig.

Die Druckschrift M. Schüler ET AL: "A novel approach for detecting HMDSO poisoning of metal oxide gas sensors and improving their stability by temperature cycled operation", J. Sens. Sens. Syst., 4, Seiten 305-311, 2015 offenbart ein Verfahren zum Quantifizieren einer HMDSO-Vergiftung in Metalloxid-Gassensoren unter Verwendung eines Temperaturzyklusbetriebs. Dadurch wird ein Verfahren zur Früherkennung von HMDSO-Vergiftungen bereitgestellt, um eine Vergiftung zu erkennen, bevor die Sensorfunktion stark beeinträchtigt ist.

Die Druckschrift M. Schüler ET AL: "Detecting poisoning of metal oxide gas sensors at an early stage by temperature cycled operation", AMA Conferences 2015, Sensor 2015 and IRS2 2015, Seiten 735- 739 offenbart die Bestimmung einer Vergiftungsdosis unter Verwendung von im Temperaturzyklusbetrieb aufgezeichneten Daten, wodurch eine einfache Methode zur frühzeitigen Erkennung v on HMDSO-Vergiftungen bereitgestellt wird.

Es ist Aufgabe der vorliegenden Erfindung, ein Verfahren zur Erkennung und Kompensation einer Degradation eines Gassensorsystems bereitzustellen, das zuverlässig die alterungs- und lagerungsbedingten Änderungen der Sensitivität des Gassensors ausgleichen kann.

Offenbarung der Erfindung

Diese Aufgabe wird durch das Verfahren zum Betreiben eines Gassensorsystems gemäß Anspruch 1 sowie eine entsprechende Vorrichtung und ein Gassensorsystem gemäß den nebengeordneten Ansprüchen gelöst.

Weitere Ausgestaltungen sind in den abhängigen Ansprüchen angegeben.

Gemäß einem ersten Aspekt ist ein Verfahren zum Betreiben eines Gassensorsystems mit einem Gassensor mit aktiver Sensorfläche in einem Diagnosebetrieb und in einem Messbetrieb vorgesehen, wobei in dem Diagnosebetrieb folgende Schritte ausgeführt werden:

- Betreiben des Gassensors in einer Hochtemperaturphase, wobei die Hochtemperaturphase bei einer Temperatur stattfindet, bei der ein Anbinden von Sauerstoffionen an Oberflächenstellen der aktiven Sensorfläche bewirkt wird;
- Erfassen einer während der Hochtemperaturphase auftretenden Änderung einer elektrischen Messgröße des Gassensors, die eine Oxidationsrate einer Anreicherung der aktiven Sensorfläche mit Sauerstoffionen angibt;
- Bestimmen einer Degradation des Gassensors abhängig von dem Verlauf oder der Änderung der elektrischen Messgröße des Gassensors;

wobei in dem Messbetrieb das Gassensorsystem abhängig von der Degradation betrieben wird.

Es ist vorgesehen, dass abhängig von der Degradation eine Korrekturgröße zum Kalibrieren des Gassensors und zum Ermitteln einer Gaskonzentration einer zu detektierenden Gaskomponente für nachfolgende Messungen der Gaskonzentration im Messbetrieb bereitgestellt wird.

Weiterhin kann im Messbetrieb des Gassensors die Korrekturgröße zum Beaufschlagen einer Reduktionsrate, die ein Maß der Reaktion der zu detektierenden Gaskomponente mit Sauerstoffionen auf der aktiven Sensorfläche angibt, oder zum Beaufschlagen einer zur Bestimmung einer Gaskonzentration erhaltenen Sensorgröße des Messbetriebs verwendet werden.

Gemäß dem obigen Verfahren wird ein Gassensorsystem betrieben, indem die katalytische Aktivität der Sensorfläche, d.h. die Fähigkeit der Sensorfläche Sauerstoffionen zu binden und in Reaktion mit den zu detektierenden Gaskomponenten freizugeben, als Maß einer Degradation gemessen wird. Damit kann die Aktivität der Sensorfläche bestimmt werden. Durch Quantifizierung der Aktivität der Sensorfläche kann eine entsprechende Änderung aufgrund Alterung und/oder einer Beaufschlagung mit Sensorschadstoffen erkannt und kompensiert werden.

Die Aktivität der Sensorfläche bestimmt maßgeblich die Sensitivität des Gassensors, und damit auch die Genauigkeit des Messsignals. Mit anderen Worten, die Sensitivität wird maßgeblich von der Reaktion der zu detektierenden Gaskomponente des umgebenden Messgases mit auf der Sensorfläche des Gassensors befindlichen Sauerstoffionen und von der Erzeugung neuer reaktiver Sauerstoffionen auf der Sensorfläche beeinflusst.

Gassensoren mit aktiver Sensorfläche können im Gleichgewichtsbetrieb oder im temperaturzyklischen Betrieb betrieben werden. In beiden Fällen wird eine elektrische Sensorgröße des Gassensors bestimmt, durch die die gemessene Gaskonzentration ermittelbar ist. Die elektrische Sensorgröße kann beispielsweise einer elektrischen Leitfähigkeit entsprechen.

Im Gleichgewichtsbetrieb wird der Gleichgewichtszustand der Reaktion der Gaskomponente mit Sauerstoffionen und die Erzeugung reaktiver Sauerstoffionen durch die Aktivität der Sensorfläche bestimmt. Je nach Konzentration der Gaskomponente in dem Messgas verschiebt sich dieser Gleichgewichtszustand und führt zu einem entsprechenden Messsignal. Verändert sich die Aktivität der Sensorfläche, so führt dies zu einem veränderten Messsignal bei gleichen Gaskonzentrationen der zu detektierenden Gaskomponente.

Bei einem temperaturzyklischen Betrieb des Gassensors wird die Sensorfläche in einer Hochtemperaturphase bei einer hohen Temperatur von ca. 350 bis 500°C mit Sauerstoffionen beladen und in einer nachfolgenden Messphase, die einen schnellen Temperatursprung auf eine deutlich niedrigere Temperatur von bis zu 300°C vorsieht, mit dem Messgas beaufschlagt. Zu Beginn der Messphase herrscht ein starkes Ungleichgewicht der Oberflächenbedeckung mit Sauerstoffionen, so dass die Reduktion mit der zu messenden Gaskomponente im Messgas dominiert. Durch Reaktion der Sauerstoffionen mit der zu detektierenden Gaskomponente kommt es zu einer Abnahme der reaktiven Sauerstoffionen an der Sensoroberfläche, die sich in einer zeitlichen Änderung des Werts der resultierenden elektrischen Sensorgröße widerspiegelt.

Durch die Möglichkeit des mehrphasigen Betriebs des Gassensors mit einer Hochtemperaturphase und einer Messphase ist es möglich, die beiden Prozesse separat voneinander zu messen. Da die Sauerstoffkonzentration in der Umgebung des Gassensors näherungsweise konstant ist, kann die Oxidationsrate der Sauerstoffionen während der Hochtemperaturphase verwendet werden, um ein Maß der Aktivität der Sensorfläche zu bestimmen. Mit dem Maß der Aktivität kann ein von der zu messenden Gaskomponente abhängiges Messsignal des Gassensors, das aus der Reaktionsrate der Gaskomponente des Messgases mit an der Sensorfläche bestimmt wird, korrigiert werden und dadurch eine alterungs- und lagerungsbedingte Degradation ausgeglichen werden.

Das obige Verfahren macht sich den Effekt zunutze, dass nach dem schnellen Temperatursprung auf die Temperatur der Hochtemperaturphase die Oxidation von Sauerstoff auf Oberflächenplätze der Sensorfläche dominiert, deren Geschwindigkeit direkt durch die Änderung der elektrischen Sensorgröße, insbesondere der elektrischen Leitfähigkeit, gemessen werden kann. Die Rate dieser Reaktion zu Beginn der Hochtemperaturphase stellt ein Maß für die Aktivität der Sensorfläche und damit ein Maß für die Degradation, d.h. einer Alterung und/oder Vergiftung, des Gassensors dar. Diese Rate bestimmt ein Maß der Degradation und kann dann verwendet werden, um eine Kompensation bzw. Korrektur des Messsignals vorzunehmen, so dass man auch bei einem Gassensor mit degradierter Sensorfläche die Gaskonzentration aus dem Messsignal mit hinreichender Genauigkeit erhalten kann.

Das obige Verfahren beruht auf der Erkenntnis, dass eine Degradation, d.h. eine Alterung bzw. Vergiftung, eines Gassensors lediglich die Geschwindigkeit aller Oberflächenprozesse, die zum Messsignal führen, verändert. Dies ermöglicht, dass das obige Verfahren die Vergiftung des Gassensors nicht nur erkennt, sondern auch mithilfe eines physikalischen Modells kompensieren kann. Dadurch ist es möglich, ein Gassensorsystem zu schaffen, das eine zuverlässige Angabe einer Gaskonzentration auch bei einem gealterten Gassensor oder einer beginnenden Sensorvergiftung ermöglicht, da eine Nachjustierung bzw. Kalibrierung des Gassensors jederzeit während des laufenden Betriebs möglich ist.

Weiterhin kann vor dem Betreiben des Gassensors in der Hochtemperaturphase eine ausreichende Menge von Sauerstoffionen auf der aktiven Sensorfläche abgebaut werden, insbesondere durch Beaufschlagen der aktiven Sensorfläche mit der zu detektierenden Gaskomponente, wobei insbesondere die ausreichende Menge von abgebauten Sauerstoffionen dadurch bestimmt ist, dass eine Änderung der elektrischen Messgröße während einer Anlagerung von Sauerstoffionen während der Hochtemperaturphase messbar ist. Eine ausreichende Menge ist somit durch die Genauigkeit der Messung des Messsignals bestimmt.

Die Menge lässt sich zudem mit Hilfe der Aktivierungsenergie des Ladungstransports messen, wie in Baur, T., Schütze, A. & Sauerwald, T., "Detektion von kurzen Gaspulsen für die Spurengasanalytik" tm - Tech. Mess. 84, 88-92 (2017) beschrieben.

Es kann vorgesehen sein, dass die Korrekturgröße einem Quotienten zwischen der bestimmten Oxidationsrate $k_{Sauerstoff}$ und einer ursprünglichen Oxidationsrate $k_{Sauerstoff,initial}$ entspricht oder diesem zugeordnet wird.

Weiterhin kann die Korrektur insbesondere durch Multiplikation einer in der Messphase erhaltenen Sensorgröße oder einer Reduktionsrate mit der Korrekturgröße erfolgen.

Gemäß einer weiteren Ausführungsform sieht das Verfahren folgende weitere Schritte vor:

- Messen einer Sensorgröße, insbesondere einer elektrischen Leitfähigkeit, in dem Messbetrieb;
- Ermitteln einer Gaskonzentration einer Gaskomponente in einem Messgas abhängig von der gemessenen Sensorgröße und der Korrekturgröße.

Es kann vorgesehen sein, dass im Messbetrieb die Gaskonzentration mithilfe der durch die Korrekturgröße korrigierten Reduktionsrate oder der durch die Korrekturgröße korrigierten elektrischen Sensorgröße basierend auf einer vorgegebenen Zuordnungsfunktion, die der elektrischen Sensorgröße und/oder der Reduktionsrate eine entsprechende Gaskonzentration zuordnet, insbesondere basierend auf einer Lookup-Tabelle, bestimmt wird.

Insbesondere kann die Zuordnungsfunktion die elektrische Sensorgröße und/oder die Reduktionsrate die entsprechende Gaskonzentration abhängig von einer Messtemperatur zuordnen.

Es kann vorgesehen sein, dass die Degradation des Gassensors, insbesondere akustisch oder optisch signalisiert wird, insbesondere bei Überschreiten eines vorgegebenen Schwellenwerts eines Maßes der Degradation.

Gemäß einer Ausführungsform kann in dem Messbetrieb eine elektrische Sensorgröße, insbesondere eine elektrische Leitfähigkeit, zur Bestimmung einer Gaskonzentration einer zu detektierenden Gaskomponente in einem Gleichgewichtsbetrieb ermittelt werden, wobei die Korrekturgröße zur Beaufschlagung der elektrischen Sensorgröße des Gleichgewichtsbetriebs bestimmt wird.

Es kann vorgesehen sein, dass der Diagnosebetrieb regelmäßig, zu vorgegebenen Zeitpunkten oder dann durchgeführt wird, wenn die gemessene Oxidationsrate von der initialen Oxidationsrate um mehr als einen vorgegebenen Abweichungswert abweicht.

Gemäß einem weiteren Aspekt ist ein Gassensorsystem mit einem Gassensor, einer Heizeinrichtung und einer Steuereinheit vorgesehen, die ausgebildet ist, den Gassensor in einem Diagnosebetrieb und in einem Messbetrieb zu betreiben, wobei die Steuereinheit ausgebildet ist, um in dem Diagnosebetrieb folgende Schritte auszuführen:

- Aufheizen des Gassensors mithilfe der Heizeinrichtung, um den Gassensor in einer Hochtemperaturphase zu betreiben, wobei die Hochtemperaturphase bei einer Temperatur stattfindet, bei der ein Anbinden von Sauerstoffionen an Oberflächenstellen der aktiven Sensorfläche bewirkt wird;
- Erfassen einer während der Hochtemperaturphase auftretenden Änderung einer elektrischen Messgröße des Gassensors, insbesondere einer elektrischen Leitfähigkeit;
- Bestimmen einer Degradation abhängig von dem Verlauf oder der Änderung der elektrischen Messgröße des Gassensors; und

wobei die Steuereinheit ausgebildet ist, um in dem Messbetrieb den Gassensor abhängig von der Degradation zu betreiben.

Die Steuereinheit kann weiterhin ausgebildet sein, um im Messbetrieb folgende Schritte auszuführen:

- Bestimmen einer Korrekturgröße abhängig von dem Verlauf oder der Änderung der elektrischen Messgröße im Diagnosebetrieb des Gassensors bzw. abhängig von der Degradation;
- Beaufschlagen der aktiven Sensorfläche mit Messgas und Messen einer elektrischen Sensorgröße des Gassensors bei einem nichttemperaturzyklischen Betrieb oder in einem Gleichgewichtsbetrieb;
- Ermitteln einer Gaskonzentration einer Gaskomponente in dem Messgas abhängig von der im Messbetrieb gemessenen Sensorgröße und der Korrekturgröße, insbesondere mithilfe einer vorgegebenen Zuordnungsfunktion, die der elektrischen Sensorgröße und/oder der Reduktionsrate eine entsprechende Gaskonzentration zuordnet.

Kurzbeschreibung der Zeichnungen

Ausführungsformen werden nachfolgend anhand der beigefügten Zeichnungen näher erläutert. Es zeigen:

Figur 1    eine schematische Darstellung eines Gassensorsystems mit einem Gassensor, der in einem temperaturzyklischen Betrieb betrieben wird; und

Figur 2    ein Flussdiagramm zur Veranschaulichung eines Verfahrens zum Betreiben des Gassensorsystems, insbesondere zur Kalibrierung des Gassensors.

Beschreibung von Ausführungsformen

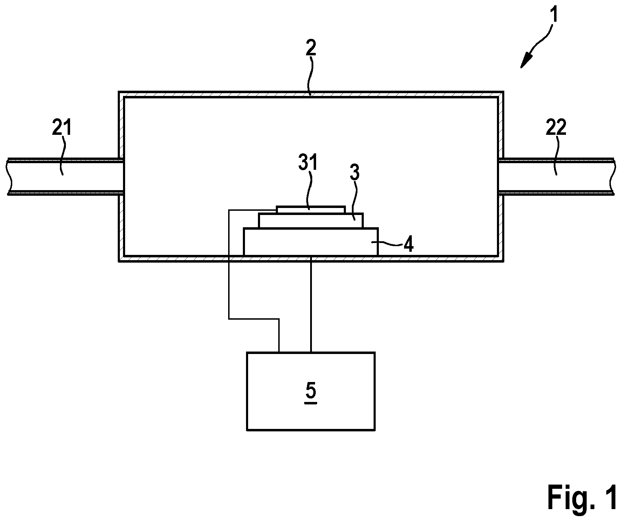

Figur 1 zeigt ein Gassensorsystem 1 mit einem in einer Messkammer 2 angeordneten Gassensor 3. Messgas wird über eine Gaszuleitung 21 der Messkammer 2 zugeführt und über eine Abführungsleitung 22 abgeführt.

Der Gassensor 3 kann als Halbleitergassensor ausgebildet sein und weist eine aktive Sensorfläche 31 zur Detektion einer Gaskonzentration einer oder mehreren im Messgas enthaltenen Gaskomponenten auf.

Der Gassensor 3 ist mit einer Heizeinrichtung 4 gekoppelt, die ein Aufheizen des Gassensors 3 auf eine hohe Temperatur von ca. 400-450°C während einer Hochtemperaturphase ermöglicht.

Die Messkammer 2 weist ein Gehäuse auf, das als Wärmesenke dient, so dass ein schnelles Abkühlen des Gassensors 3 bei deaktivierter Heizeinrichtung 4 möglich ist.

Es ist eine Steuereinheit 5 vorgesehen, die den Betrieb des Gassensors 3 steuert. Der Gassensor 3 wird im gezeigten Ausführungsbeispiel in einem temperaturzyklischen Betrieb betrieben, mit der Hochtemperaturphase, bei der der Gassensor auf eine hohe Temperatur von zwischen 400 bis 450°C aufgeheizt wird. Die Hochtemperaturphase wird für eine vorbestimmte Zeitdauer gehalten, so dass eine Aufladung der aktiven Sensorfläche des Gassensors mit Sauerstoffionen erfolgt. Nach der Hochtemperaturphase erfolgt, gesteuert durch die Steuereinheit 5, ein Temperatursprung durch Abschalten der Heizeinrichtung 4 auf eine deutlich niedrigere Temperatur in einer Niedertemperaturphase von 100 bis 300°C. Diese entspricht einer Messphase.

Bei Beginn der Messphase herrscht ein starkes Ungleichgewicht der Oberflächenbedeckung mit Sauerstoffionen, so dass Reaktionen mit der zu detektierenden Gaskomponente in dem durch die Messkammer 2 strömenden Messgas dominieren. Diese Reaktion mit den Sauerstoffionen führt zu einer Leitfähigkeitsänderung (Änderung der elektrischen Sensorgröße) des Gassensors 3, die zur Bestimmung der Gaskonzentration der Gaskomponente in dem Messgas charakteristisch ist bzw. verwendet wird.

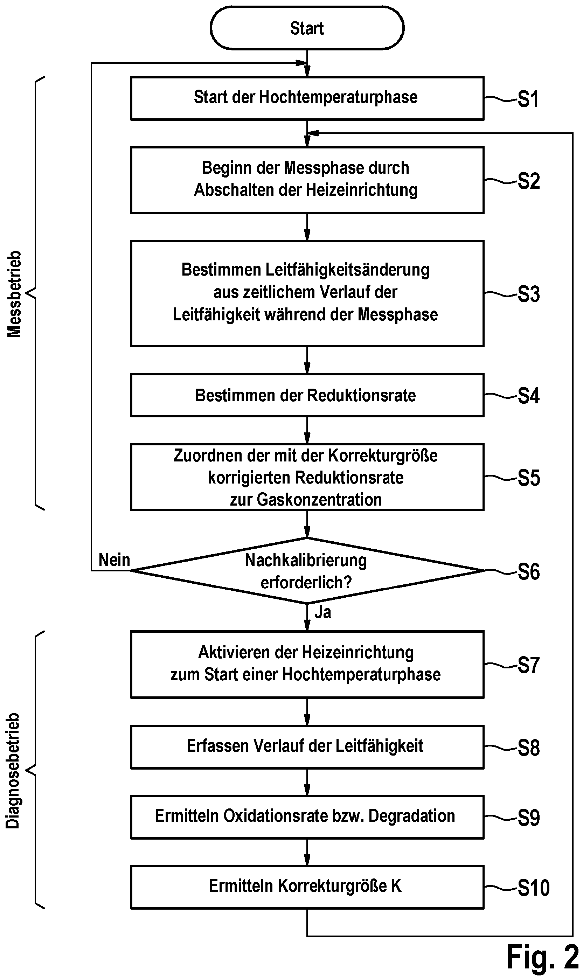

In Figur 2 ist ein Flussdiagramm zur Veranschaulichung eines Verfahrens zum Betreiben des Gassensorsystems 1 im temperaturzyklischen Betrieb beschrieben. Das Verfahren wird durch die Steuereinheit 5 gesteuert, in der dieses Verfahren als Software oder Hardwarealgorithmus implementiert ist

Das Verfahren beginnt in einem Messbetrieb. Dazu wird in Schritt S1 der Gassensor 3 während der Hochtemperaturphase aufgeheizt, um die Sensorfläche 31 zu aktivieren. Die Aufheizung in der Hochtemperaturphase wird während einer vorbestimmten Zeitdauer von 0,1 bis 60 s beibehalten.

Anschließend wird in Schritt S2 die Heizeinrichtung 4 zum Starten der Messphase abgeschaltet bzw. zum Einstellen oder Einregeln einer neuen niedrigeren Temperaturvorgabe betrieben, so dass der Gassensor einen Temperatursprung auf eine deutlich niedrigere Temperatur erfährt. Zu Beginn der Messphase herrscht ein starkes Ungleichgewicht der Oberflächenbedeckung mit Sauerstoff, so dass die zu messenden Gaskomponenten mit den Sauerstoffionen reagieren, was zu einer Änderung einer elektrischen Sensorgröße, insbesondere zu einer Änderung der elektrischen Leitfähigkeit G, des Gassensors 3 führt.

Das Maß der Änderung der Leitfähigkeit kann in dem Schritt S3 bestimmt werden. Dazu wird ab Beginn der Messphase ein zeitlicher Verlauf der Leitfähigkeit (Sensorgröße) erfasst und durch Gradientenbildung ein Gradient der Leitfähigkeitsänderung (Änderung der Sensorgröße) ermittelt.

Im Schritt S4 wird entsprechend einem Funktionsmodell $k_{gas,LT}(c)$ bestimmt. Das Funktionsmodell kann gemäß folgender Beziehung physikalisch motiviert sein:

$$\frac{dlnG}{dt} = \frac{2 \cdot E_B(0)}{k_B T} \cdot \left(-k_{Sauerstoff,LT} + k_{gas,LT}(c)\right)$$

wobei G der Leitfähigkeit, $E_B(0)$ der Energiebarriere als Materialeigenschaft des Gassensors 3, $k_B$ der Boltzmann-Konstante, t Zeitpunkten eines zeitlichen Verlaufs der Messung, T der Temperatur des Gassensors 3, $k_{Sauerstoff}$ der Oxidationsrate mit Sauerstoffionen, die auf niedriger Temperatur zu vernachlässigen ist ($k_{Sauerstoff,LT} = 0$), und $k_{gas,LT}(c)$ die von der Gaskonzentration c der Gaskomponente im Messgas abhängige Reduktionsrate entsprechen.

Alternativ kann das Funktionsmodell auch als mathematisch gefittetes Modell bereitgestellt werden:

$$lnG(t) = a \cdot e^{-b \cdot t} + c$$

Die Parameter a, b, c werden durch Anfitten basierend auf Messungen während der Hochtemperaturphase ermittelt. Dabei kann als Angabe für die Oxidationsrate $\frac{1}{b}$ angenommen werden, was etwa proportional zur Oxidationsrate $k_{Sauerstoff,HT}$ ist.

Welches der Funktionsmodelle Anwendung findet, hängt unter anderem davon ab, wie schnell das Sensorsignal sich ändert und welche zeitliche Auflösung die verwendete Elektronik hat.

Zur Berücksichtigung einer Korrektur durch eine Korrekturgröße K aufgrund der Degradation der Sensitivität des Gassensors 3 kann die obige Formel wie folgt präzisiert werden:

$$\frac{dlnG}{dt} = \frac{2 \cdot E_B(0)}{k_B T} \cdot \left(-k_{Sauerstoff,LT} + K k_{gas,LT,mess}(c)\right)$$

Zur Ermittlung der Gaskonzentration c kann in Schritt S5 die bestimmte durch vorstehende Formel korrigierte Reduktionsrate $K k_{gas,LT,mess}(c)$ einem Gaskonzentrationswert c zugeordnet werden, insbesondere mithilfe einer vorgegebenen Zuordnungsfunktion, wie z.B. einer Lookup-Tabelle. Dabei wird angenommen, dass die Sauerstoffkonzentrationen während der Hochtemperaturphase und der Messphase konstant und identisch sind und keinen Einfluss auf die Oxidationsrate hat.

Alternativ oder zusätzlich kann die Degradation des Gassensors 3 signalisiert werden. Dies kann insbesondere dadurch erfolgen, dass durch eine optische oder akustische Anzeige die Notwendigkeit eines Austausches des Gassensors 3 angegeben wird. Dies kann insbesondere dann vorgenommen werden, wenn das Maß der Degradation einen vorgegebenen Schwellenwert übersteigt.

In Schritt S6 wird überprüft, ob eine Nachkalibrierung des Gassensorsystems 1 erforderlich ist. Dies kann beispielsweise festgestellt werden, indem eine vorbestimmte Zeitdauer seit der letzten Nachkalibrierung vergangen ist. Weiterhin kann dies festgestellt werden, wenn die gemessene Oxidationsrate $k_{Sauerstoff,HT}$ von der initialen Oxidationsrate $k_{Sauerstoff,initial,HT}$ um mehr als einen vorgegebenen Abweichungswert abweicht. Die Wahl des Abweichungswerts ist abhängig von der Anwendung (Sicherheitsrelevant...). Wird festgestellt, dass eine Nachkalibrierung durchgeführt werden soll, wird das Verfahren mit Schritt S7 in einem Diagnosebetrieb fortgesetzt, anderenfalls wird zu Schritt S1 zurückgesprungen. Der Diagnosebetrieb dient zur Ermittlung einer Degradation bzw. einer Korrekturgröße K für die Messung im Messbetrieb.

In Schritt S7 wird durch Aktivieren der Heizeinrichtung 4 eine Hochtemperaturphase des Diagnosebetriebs eingeleitet.

Dabei wird der Verlauf der elektrischen Sensorgröße G (elektrische Leitfähigkeit) in Schritt S8 erfasst und in Schritt S9 entsprechend des in Schritt S4 verwendeten Funktionsmodells

$$\frac{dlnG}{dt} = \frac{2 \cdot E_B(0)}{k_B T} \cdot \left(-k_{Sauerstoff,HT}\right)$$

oder

$$lnG(t) = a \cdot e^{-b \cdot t} + c \text{ , wobei } \frac{1}{b} \propto k_{Sauerstoff,HT}$$

die Oxidationsrate $k_{Sauerstoff,HT}$ ermittelt. Dabei kann in obiger Formel die Reduktionsrate $k_{gas,HT}$ der Hochtemperaturphase vernachlässigt werden.

Die Oxidationsrate $k_{Sauerstoff,HT}$ während der Hochtemperaturphase stellt ein Maß für die Degradation des Gassensors 3 bzw. die Degradation dar.

In Schritt S10 kann dann eine Korrekturgröße K ermittelt werden, die die Alterung bzw. die Sensorvergiftung des Gassensors 3 angibt:

$$k_{gas,korr} = \left( \frac{k_{Sauerstoff,initial,HT}}{k_{Sauerstoff,HT}} \cdot a \cdot k_{gas,mess} \right)$$

$$K = \frac{k_{Sauerstoff,initial,HT}}{k_{Sauerstoff,HT}} \cdot a$$

wobei die Konstante a für den Typ des Gassensorsystems 1 und die zu messenden Gase/Gasgemische bestimmt wird.

Dabei kann die in dem Schritt S4 ermittelte Reduktionsrate $k_{gas,LT,mess}$ korrigiert werden durch einen Korrekturwert der Korrekturgröße K, der einem Quotienten zwischen der Oxidationsrate $k_{Sauerstoff,HT}$ während der Hochtemperaturphase und der ursprünglichen/initialen Oxidationsrate $k_{Sauerstoff,initial,HT}$ während der Hochtemperaturphase bei Inbetriebnahme des Gassensorsystems 1 entspricht.

Anschließend wird das Verfahren mit dem Start der Messphase in Schritt S2 fortgesetzt.

Um eine Bestimmung des Korrekturwerts für die Reduktionsrate notwendige Abbau der Sauerstoffionen auf der Sensoroberfläche während der Hochtemperaturphase zu gewährleisten, ist es erforderlich, dass eine anschließende Ruhephase oder die anschließende Messphase ausreichend lange ist, so dass die Gaskomponente in dem Messgas in ausreichendem Maße die Sauerstoffionen der Sensorfläche 31 abgebaut hat. Somit kann als eine Bedingung für das Durchführen der Nachkalibrierung in Schritt S6 eine ausreichende hohe zuletzt gemessene Konzentration der Gaskomponente angenommen werden.

Erkennen kann man einen ausreichenden Abbau der Sauerstoffionen an der Sensorfläche 31 beispielsweise dadurch, dass eine Schwellwertbetrachtung bezüglich des Leitwerts durchgeführt wird. Beispielsweise kann eine ausreichende Dauer der Ruhephase/Messphase erkannt werden, wenn der Leitwert zu Beginn der Ruhephase / Messphase sich um einen vorgegeben relativen oder absoluten Betrag erhöht hat, bevor ein Umschalten zu der Hochtemperaturphase zugelassen wird.

Das obige Verfahren ist auch anwendbar auf Gassensoren im Gleichgewichtsbetrieb, wobei das Verfahren jedoch eine ausreichende Wartezeit nach einer Hochtemperaturphase zur Bestimmung der Korrekturgröße K gewährleisten muss, damit sich ein Gleichgewichtszustand einstellen kann.

Bei einem Gleichgewichtsbetrieb wird eine gemäß obiger Vorgehensweise bestimmte Korrekturgröße K dabei zum Beaufschlagen der elektrischen Sensorgröße, insbesondere einer elektrischen Leitfähigkeit, die sich im Gleichgewichtszustand einstellt, im Messbetrieb verwendet, insbesondere durch Multiplikation der Korrekturgröße K mit der Sensorgröße.

**Patentansprüche**

1.  Verfahren zum Betreiben eines Gassensorsystems (1) mit einem Gassensor (3) mit aktiver Sensorfläche (31) in einem Diagnosebetrieb und in einem Messbetrieb, wobei in dem Diagnosebetrieb folgende Schritte ausgeführt werden:

    - Betreiben (S7) des Gassensors (3) in einer Hochtemperaturphase, wobei die Hochtemperaturphase bei einer Temperatur stattfindet, bei der ein Anbinden von Sauerstoffionen an Oberflächenstellen der aktiven Sensorfläche (31) bewirkt wird;
    - Erfassen (S8) einer während der Hochtemperaturphase auftretenden Änderung einer elektrischen Messgröße, insbesondere einer elektrischen Leitfähigkeit, des Gassensors (3), die eine Oxidationsrate einer Anreicherung der aktiven Sensorfläche (31) mit Sauerstoffionen angibt;
    - Bestimmen (S9) einer Degradation des Gassensors (3) abhängig von der Änderung der elektrischen Messgröße des Gassensors (3);
    wobei in dem Messbetrieb das Gassensorsystem (1) abhängig von der Degradation betrieben wird,

**dadurch gekennzeichnet, dass** abhängig von der Degradation eine Korrekturgröße (K) zum Kalibrieren des Gassensors (3) und zum Ermitteln einer Gaskonzentration (c) einer zu detektierenden Gaskomponente für nachfolgende Messungen der Gaskonzentration im Messbetrieb bereitgestellt wird.

2. Verfahren nach Anspruch 1, wobei vor dem Betreiben des Gassensors (3) in der Hochtemperaturphase eine ausreichende Menge von Sauerstoffionen auf der aktiven Sensorfläche (31) abgebaut wird, insbesondere durch Beaufschlagen der aktiven Sensorfläche (31) mit der zu detektierenden Gaskomponente, wobei insbesondere die ausreichende Menge von abgebauten Sauerstoffionen dadurch bestimmt ist, dass eine Änderung der elektrischen Messgröße während einer Anlagerung von Sauerstoffionen während der Hochtemperaturphase messbar ist.

3. Verfahren nach Anspruch 2, wobei im Messbetrieb des Gassensors (3) die Korrekturgröße (K) zum Beaufschlagen einer Reduktionsrate, die ein Maß der Reaktion der zu detektierenden Gaskomponente mit Sauerstoffionen auf der aktiven Sensorfläche (31) angibt, oder einer zur Bestimmung einer Gaskonzentration erhaltenen Messgröße (G) verwendet wird.

4. Verfahren nach einem der Ansprüche 2 bis 3, wobei die Korrekturgröße (K) einem Quotienten zwischen der bestimmten Oxidationsrate ($k_{Sauerstoff}$) und einer ursprünglichen Oxidationsrate ($k_{Sauerstoff,initial}$) entspricht oder diesem zugeordnet wird.

5. Verfahren nach Anspruch 4, wobei die Korrektur insbesondere durch Multiplikation einer in der Messphase erhaltenen Sensorgröße (G) oder einer Reduktionsrate mit der Korrekturgröße (K) erfolgt.

6. Verfahren nach einem der Ansprüche 2 bis 5, mit folgenden Schritten:

   - Messen einer Sensorgröße in dem Messbetrieb;
   - Ermitteln einer Gaskonzentration (c) einer Gaskomponente in einem Messgas abhängig von der gemessenen Sensorgröße und der Korrekturgröße (K).

7. Verfahren nach Anspruch 6, wobei im Messbetrieb die Gaskonzentration mithilfe der durch die Korrekturgröße (K) korrigierten Reduktionsrate oder der durch die Korrekturgröße (K) korrigierten elektrischen Sensorgröße des Messbetriebs basierend auf einer vorgegebenen Zuordnungsfunktion, die der elektrischen Sensorgröße (G) und/oder der Reduktionsrate eine entsprechende Gaskonzentration zuordnet, insbesondere basierend auf einer Lookup-Tabelle, bestimmt wird.

8. Verfahren nach Anspruch 7, wobei die Zuordnungsfunktion die elektrische Sensorgröße und/oder die Reduktionsrate die entsprechende Gaskonzentration (c) abhängig von einer Messtemperatur zuordnet.

9. Verfahren nach einem der Ansprüche 1 bis 8, wobei die Degradation des Gassensors (3), insbesondere akustisch oder optisch, signalisiert wird, insbesondere bei Überschreiten eines vorgegebenen Schwellenwerts eines Maßes der Degradation des Gassensors (3).

10. Verfahren nach einem der Ansprüche 1 bis 9, wobei in dem Messbetrieb eine elektrische Messgröße, insbesondere einer elektrischen Leitfähigkeit, in einem Gleichgewichtsbetrieb ermittelt wird, wobei die Korrekturgröße (K) zur Beaufschlagung der elektrischen Sensorgröße, die sich im Gleichgewichtsbetrieb einstellt, bestimmt wird.

11. Verfahren nach einem der Ansprüche 1 bis 10, wobei der Diagnosebetrieb regelmäßig, zu vorgegebenen Zeitpunkten oder dann durchgeführt wird, wenn die gemessene Oxidationsrate ($k_{Sauerstoff,HT}$) von der initialen Oxidationsrate ($k_{Sauerstoff,initiai,HT}$) um mehr als einen vorgegebenen Abweichungswert abweicht.

12. Gassensorsystem (1) mit einem Gassensor (3), einer Heizeinrichtung (4) und einer Steuereinheit (5), die ausgebildet ist, den Gassensor (3) in einem Diagnosebetrieb und in einem Messbetrieb zu betreiben, wobei die Steuereinheit (5) ausgebildet ist, um in dem Diagnosebetrieb folgende Schritte auszuführen:

   - Betreiben des Gassensors (3) in einer Hochtemperaturphase, wobei die Hochtemperaturphase bei einer Temperatur stattfindet, bei der ein Anbinden von Sauerstoffionen an Oberflächenstellen der aktiven Sensorfläche (31) bewirkt wird;
   - Erfassen einer während der Hochtemperaturphase auftretenden Änderung einer elektrischen Messgröße des Gassensors (3), die eine Oxidationsrate einer Anreicherung der aktiven Sensorfläche (31) mit Sauerstoffionen angibt;
- Bestimmen einer Degradation des Gassensors (3) abhängig von der Änderung der elektrischen Messgröße des Gassensors (3);
wobei die Steuereinheit (5) ausgebildet ist, um in dem Messbetrieb das Gassensorsystem (1) abhängig von der Degradation zu betreiben,
**dadurch gekennzeichnet, dass** abhängig von der Degradation eine Korrekturgröße (K) zum Kalibrieren des Gassensors (3) und zum Ermitteln einer Gaskonzentration (c) einer zu detektierenden Gaskomponente für nachfolgende Messungen der Gaskonzentration im Messbetrieb bereitgestellt wird.

13. Gassensorsystem (1) nach Anspruch 12, wobei die Steuereinheit (5) weiterhin ausgebildet ist, um im Messbetrieb folgende weitere Schritte auszuführen:

- Beaufschlagen der aktiven Sensorfläche (31) mit Messgas und Messen einer elektrischen Sensorgröße (G) des Gassensors (3) bei einem nichttemperaturzyklischen Betrieb oder eines Verlaufs der elektrischen Sensorgröße (G) bei einem temperaturzyklischen Betrieb;
- Ermitteln einer Gaskonzentration einer Gaskomponente in dem Messgas abhängig von der im Messbetrieb gemessenen Sensorgröße(n) und der Korrekturgröße (K).

**Claims**

1. Method for operating a gas sensor system (1) having a gas sensor (3) with an active sensor surface (31) in a diagnostic mode and in a measuring mode, the following steps being carried out in the diagnostic mode:

   - operating (S7) the gas sensor (3) in a high-temperature phase, the high-temperature phase taking place at a temperature at which oxygen ions are caused to bind to surface points of the active sensor surface (31);
   - detecting (S8) a change in an electrical measured variable, in particular an electrical conductivity, of the gas sensor (3), which change occurs during the high-temperature phase and indicates an oxidation rate of an enrichment of the active sensor surface (31) with oxygen ions;
   - determining (S9) a degradation of the gas sensor (3) as a function of the change in the electrical measured variable of the gas sensor (3);
   wherein, in the measuring mode, the gas sensor system (1) is operated as a function of the degradation, **characterized in that** a correction variable (K) for calibrating the gas sensor (3) and for determining a gas concentration (c) of a gas component to be detected is provided for subsequent measurements of the gas concentration in the measuring mode as a function of the degradation.

2. Method according to claim 1, wherein a sufficient quantity of oxygen ions on the active sensor surface (31) is reduced in the high temperature phase before operating the gas sensor (3), in particular by applying the active sensor surface (31) with the gas component to be detected, wherein in particular the sufficient quantity of reduced oxygen ions is determined by measuring a change in the electrical measured variable during an accumulation of oxygen ions during the high temperature phase.

3. A method according to claim 2, wherein, in the measuring mode of the gas sensor (3), the correction variable (K) is used to apply a reduction rate which indicates a measure of the reaction of the gas component to be detected with oxygen ions on the active sensor surface (31), or a measured variable (G) obtained to determine a gas concentration.

4. A method according to one of claims 2 to 3, wherein the correction variable (K) corresponds to a quotient between the determined oxidation rate ($k\_oxygen$) and an original oxidation rate ($k_{(oxygen,initial)}$) or is assigned to it.

5. A method according to claim 4, wherein the correction is carried out in particular by multiplying a sensor variable (G) obtained in the measuring phase or a reduction rate by the correction variable (K).

6. A method according to any one of claims 2 to 5, comprising the following steps:

   - measuring a sensor variable in the measuring mode;
   - determining a gas concentration (c) of a gas component in a measuring gas as a function of the measured sensor variable and the correction variable (K).

7. Method according to claim 6, wherein in the measuring mode the gas concentration is determined with the aid of the reduction rate corrected by the correction variable (K) or the electrical sensor variable of the measuring mode corrected by the correction variable (K) based on a predetermined assignment function, which assigns a corresponding gas concentration to the electrical sensor variable (G) and/or the reduction rate, in particular based on a lookup table.

8. Method according to claim 7, wherein the assignment function assigns the electrical sensor variable and/or the reduction rate to the corresponding gas concentration (c) as a function of a measurement temperature.

9. A method according to one of claims 1 to 8, wherein the degradation of the gas sensor (3) is signaled, in particular acoustically or optically, in particular when a predetermined threshold value of a measure of the degradation of the gas sensor (3) is exceeded.

10. Method according to one of claims 1 to 9, in which, in the measuring mode, an electrical measured variable, in particular an electrical conductivity, is determined in an equilibrium mode, the correction variable (K) for acting on the electrical sensor variable, which is set in the equilibrium mode, being determined.

11. Method according to one of claims 1 to 10, wherein the diagnostic mode is carried out regularly, at predetermined times or when the measured oxidation rate ($k_{(oxygen,HT)}$) deviates from the initial oxidation rate ($k_{(oxygen, initial, HT)}$) by more than a predetermined deviation value.

12. Gas sensor system (1) having a gas sensor (3), a heating device (4) and a control unit (5) which is designed to operate the gas sensor (3) in a diagnostic mode and in a measuring mode, the control unit (5) being designed to carry out the following steps in the diagnostic mode:

- operating the gas sensor (3) in a high-temperature phase, the high-temperature phase taking place at a temperature at which oxygen ions are caused to bind to surface sites of the active sensor area (31);
- detecting a change in an electrical measurement variable of the gas sensor (3) which occurs during the high-temperature phase and indicates an oxidation rate of an enrichment of the active sensor surface (31) with oxygen ions;
- determining a degradation of the gas sensor (3) as a function of the change in the electrical measured variable of the gas sensor (3);
the control unit (5) being designed to operate the gas sensor system (1) as a function of the degradation in the measuring mode,
**characterized in that**
a correction variable (K) for calibrating the gas sensor (3) and for determining a gas concentration (c) of a gas component to be detected is provided for subsequent measurements of the gas concentration in the measuring mode as a function of the degradation.

13. Gas sensor system (1) according to claim 12, wherein the control unit (5) is further designed to carry out the following further steps in the measuring mode:

- .applying measuring gas to the active sensor surface (31) and measuring an electrical sensor variable (G) of the gas sensor (3) in the case of non-temperature-cyclic operation or measuring a profile of the electrical sensor variable (G) in the case of temperature-cyclic operation;
- determining a gas concentration of a gas component in the measuring gas as a function of the sensor variable(s) measured in the measuring mode and the correction variable (K).

**Revendications**

1. Procédé de fonctionnement d'un système de capteur de gaz (1) comprenant un capteur de gaz (3) avec une surface de capteur active (31) dans un mode de diagnostic et dans un mode de mesure, les étapes suivantes étant exécutées dans le mode de diagnostic :

- faire fonctionner (S7) le capteur de gaz (3) dans une phase de haute température, la phase de haute température ayant lieu à une température à laquelle une fixation d'ions d'oxygène est effectuée à des emplacements de surface de la surface de capteur active (31) ;

- détecter (S8) une variation, survenant pendant la phase à haute température, d'une grandeur de mesure électrique, en particulier d'une conductivité électrique, du capteur de gaz (3), qui indique un taux d'oxydation d'un enrichissement de la surface active du capteur (31) en ions oxygène ;
- déterminer (S9) une dégradation du capteur de gaz (3) en fonction de la variation de la grandeur de mesure électrique du capteur de gaz (3) ;
- dans lequel, dans le mode de mesure, le système de capteur de gaz (1) est actionné en fonction de la dégradation,

**caractérisé en ce que**, en fonction de la dégradation, une grandeur de correction (K) est mise à disposition pour calibrer le capteur de gaz (3) et pour déterminer une concentration de gaz (c) d'un composant gazeux à détecter pour des mesures ultérieures de la concentration de gaz en mode de mesure.

2. Procédé selon la revendication 1, dans lequel, avant le fonctionnement du capteur de gaz (3) dans la phase à haute température, une quantité suffisante d'ions oxygène est dégradée sur la surface active du capteur (31), en particulier par l'exposition de la surface active du capteur (31) au composant gazeux à détecter, en particulier la quantité suffisante d'ions oxygène dégradés étant déterminée par le fait qu'une modification de la grandeur de mesure électrique est mesurable pendant une accumulation d'ions oxygène pendant la phase à haute température.

3. Procédé selon la revendication 2, dans lequel, en mode de mesure du capteur de gaz (3), la grandeur de correction (K) est utilisée pour appliquer un taux de réduction, qui indique une mesure de la réaction du composant gazeux à détecter avec des ions oxygène sur la surface active du capteur (31), ou une grandeur de mesure (G) obtenue pour déterminer une concentration de gaz.

4. Procédé selon l'une des revendications 2 à 3, dans lequel la grandeur de correction (K) correspond ou est associée à un quotient entre le taux d'oxydation déterminé ($k\_oxygène$) et un taux d'oxydation initial ($k\_{(oxygène,initial)}$).

5. Procédé selon la revendication 4, dans lequel la correction est notamment réalisée en multipliant une grandeur de capteur (G) ou un taux de réduction obtenu lors de la phase de mesure par la grandeur de correction (K).

6. Procédé selon l'une des revendications 2 à 5, comprenant les étapes suivantes :

   - Mesurer d'une grandeur de capteur dans le mode de mesure ;
   - Déterminer une concentration en gaz (c) d'un composant gazeux dans un gaz de mesure en fonction de la grandeur de capteur mesurée et de la grandeur de correction (K).

7. Procédé selon la revendication 6, dans lequel, en mode de mesure, la concentration de gaz est déterminée à l'aide du taux de réduction corrigé par la grandeur de correction (K) ou de la grandeur électrique de capteur du mode de mesure corrigée par la grandeur de correction (K), sur la base d'une fonction d'affectation prédéterminée qui affecte une concentration de gaz correspondante à la grandeur électrique de capteur (G) et/ou au taux de réduction, en particulier sur la base d'une table de consultation.

8. Procédé selon la revendication 7, dans lequel la fonction d'affectation affecte à la grandeur électrique du capteur et/ou au taux de réduction la concentration en gaz (c) correspondante en fonction d'une température de mesure.

9. Procédé selon l'une des revendications 1 à 8, dans lequel la dégradation du capteur de gaz (3) est signalée, notamment de manière sonore ou visuelle, notamment en cas de dépassement d'un seuil prédéterminé d'une mesure de la dégradation du capteur de gaz (3).

10. Procédé selon l'une des revendications 1 à 9, dans lequel, en mode de mesure, on détermine une grandeur électrique mesurée, notamment une conductivité électrique, en mode d'équilibre, la grandeur de correction (K) étant déterminée pour alimenter la grandeur électrique du capteur qui s'établit en mode d'équilibre.

11. Procédé selon l'une des revendications 1 à 10, dans lequel le mode diagnostic est effectué régulièrement, à des instants prédéterminés ou lorsque le taux d'oxydation mesuré ($k\_{(oxygène,HT)}$) s'écarte du taux d'oxydation initial ($k\_{(oxygène,initial,HT)}$) de plus d'une valeur d'écart prédéterminée.

12. Système de capteur de gaz (1) comprenant un capteur de gaz (3), un dispositif de chauffage (4) et une unité de commande (5) qui est conçue pour faire fonctionner le capteur de gaz (3) dans un mode de diagnostic et dans un mode de mesure, l'unité de commande (5) étant conçue pour exécuter les étapes suivantes dans le mode de diagnostic :

- faire fonctionner le capteur de gaz (3) dans une phase de haute température, la phase de haute température ayant lieu à une température à laquelle une liaison d'ions d'oxygène est provoquée aux points de surface de la surface active du capteur (31) ;
- détecter une variation d'une mesure électrique du capteur de gaz (3) se produisant pendant la phase à haute température, qui indique un taux d'oxydation d'un enrichissement de la surface active du capteur (31) avec des ions oxygène ;
- déterminer une dégradation du capteur de gaz (3) en fonction de la variation de la grandeur de mesure électrique du capteur de gaz (3) ;

l'unité de commande (5) étant conçue pour faire fonctionner le système de capteur de gaz (1) dans le mode de mesure en fonction de la dégradation, **caractérisé en ce que**, en fonction de la dégradation, une grandeur de correction (K) est mise à disposition pour calibrer le capteur de gaz (3) et pour déterminer une concentration de gaz (c) d'un composant gazeux à détecter pour des mesures ultérieures de la concentration de gaz en mode de mesure.

13. Système de détection de gaz (1) selon la revendication 12, dans lequel l'unité de commande (5) est en outre conçue pour exécuter les étapes supplémentaires suivantes en mode de mesure :

- Application d'un gaz de mesure sur la surface active (31) du capteur et mesure d'une grandeur électrique (G) du capteur de gaz (3) lors d'un fonctionnement non cyclique en fonction de la température ou d'une évolution de la grandeur électrique (G) du capteur lors d'un fonctionnement cyclique en fonction de la température ;
- déterminer une concentration de gaz d'un composant gazeux dans le gaz de mesure en fonction de la ou des grandeurs de capteur mesurées en mode de mesure et de la grandeur de correction (K).

Fig. 1

```
                    ╭─────────────────────╮
                    │        Start        │
                    ╰─────────────────────╯
                              │
   ┌─────────────────────────────────────────────────────┐
   │          Start der Hochtemperaturphase              │────S1
   └─────────────────────────────────────────────────────┘
                              │
   ┌─────────────────────────────────────────────────────┐
   │             Beginn der Messphase durch              │
   │           Abschalten der Heizeinrichtung            │────S2
   └─────────────────────────────────────────────────────┘
                              │
   ┌─────────────────────────────────────────────────────┐
   │             Bestimmen Leitfähigkeitsänderung        │
   │               aus zeitlichem Verlauf der            │────S3
   │          Leitfähigkeit während der Messphase        │
   └─────────────────────────────────────────────────────┘
                              │
   ┌─────────────────────────────────────────────────────┐
   │             Bestimmen der Reduktionsrate            │────S4
   └─────────────────────────────────────────────────────┘
                              │
   ┌─────────────────────────────────────────────────────┐
   │           Zuordnen der mit der Korrekturgröße       │
   │             korrigierten Reduktionsrate             │────S5
   │                 zur Gaskonzentration                │
   └─────────────────────────────────────────────────────┘
                              │
            Nein   ◇ Nachkalibrierung erforderlich? ◇────S6
                              │ Ja
   ┌─────────────────────────────────────────────────────┐
   │             Aktivieren der Heizeinrichtung          │
   │          zum Start einer Hochtemperaturphase        │────S7
   └─────────────────────────────────────────────────────┘
                              │
   ┌─────────────────────────────────────────────────────┐
   │            Erfassen Verlauf der Leitfähigkeit       │────S8
   └─────────────────────────────────────────────────────┘
                              │
   ┌─────────────────────────────────────────────────────┐
   │         Ermitteln Oxidationsrate bzw. Degradation   │────S9
   └─────────────────────────────────────────────────────┘
                              │
   ┌─────────────────────────────────────────────────────┐
   │              Ermitteln Korrekturgröße K             │────S10
   └─────────────────────────────────────────────────────┘
```

Messbetrieb (S1–S5)

Diagnosebetrieb (S7–S10)

Fig. 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **M. SCHÜLER et al.** A novel approach for detecting HMDSO poisoning of metal oxide gas sensors and improving their stability by temperature cycled operation. *J. Sens. Sens. Syst.*, 2015, vol. 4, 305-311

- **M. SCHÜLER et al.** Detecting poisoning of metal oxide gas sensors at an early stage by temperature cycled operation. *AMA Conferences*, 2015, 735-739
- **BAUR, T. ; SCHÜTZE, A. ; SAUERWALD, T.** Detektion von kurzen Gaspulsen für die Spurengasanalytik. *tm - Tech. Mess.*, 2017, vol. 84, 88-92